# EUROPÄISCHE PATENTANMELDUNG

(11) **EP 4 726 357 A1**
(43) Veröffentlichungstag der Anmeldung: **15.04.2026**
(21) Anmeldenummer: 25206793.9
(22) Anmeldetag: 06.10.2025
(51) Int. Cl.: G01N 11/00, G16H 50/20

(54) **MOBILE RHEOMETERVORRICHTUNG UND VERFAHREN FÜR DIE MEDINZINISCHE DIAGNOSTIK VON MENSCHLICHEN, TIERISCHEN ODER PFLANZLICHEN PROBEN**

(30) Priorität: 09.10.2024 DE 102024129203
(71) Anmelder: Netzsch-Gerätebau GmbH, 95100 Selb (DE)
(72) Erfinder: Küspert, Sabrina, 95100 Selb (DE); Rummel, Florian, 95100 Selb (DE); Szántó, Levente, 95028 Hof (DE); Taubmann, Rebekka, 95100 Selb (DE)

(57) **Zusammenfassung**

Die vorliegende Erfindung betrifft eine mobile Rheometervorrichtung (1) für die medizinische Diagnostik von menschlichen, tierischen oder pflanzlichen Proben. Solch eine mobile Rheometervorrichtung (1) umfasst dabei eine rheologische Messeinrichtung (2), eine damit verbundene Auswerteeinheit (4) mit Auswerteprogramm (5) und eine damit verbunden Ausgabeeinheit (6). Dabei ist die Auswerteeinheit (4) mit Auswerteprogramm (5) ausgelegt, die gemessenen rheologischen Parameter einer menschlichen, tierischen oder pflanzlichen Probe hinsichtlich wenigstens einer medizinischen Diagnose oder in relevante Parametergruppen für eine medizinische Diagnose zu klassifizieren, sodass einem Anwender der mobilen Rheometervorrichtung (1) eine differenzierte Ansicht der Probe mittels der Ausgabeeinheit (6) bereitstellbar ist. Zudem wird ein entsprechendes Verfahren offenbart.

## Beschreibung

### TECHNISCHES GEBIET DER ERFINDUNG

Die vorliegende Erfindung betrifft eine mobile Rheometervorrichtung sowie ein Verfahren für die medizinische Diagnostik von menschlichen, tierischen oder pflanzlichen Proben.

### HINTERGRUND DER ERFINDUNG

Um weitergehende Aussagen über menschliche, tierische oder pflanzliche Proben treffen zu können, werden diese häufig nach der Entnahme vom Organismus aufwendig im Labor analysiert. Alternativ ist auch bekannt, eine im Zusammenhang mit dem zu untersuchendem Gewebe oder Organismus stehende Flüssigkeit zu untersuchen.

So werden beispielsweise Proben von einem Menschen während einer Biopsie oder einer Operation durch medizinisches Fachpersonal entnommen und anschließend zur Pathologie gebracht. In der Pathologie erfolgt anschließend häufig eine histologische Untersuchung, typischerweise mit Färbung von Gewebe und nachfolgender pathologischer Diagnostik. Auch können Gewebeproben mittels Gewebeschnitte aufbereitet werden, um diese einzelnen Schnitte jeweils anschließend weiter zu analysieren.

Anstatt einer histologischen Untersuchung mit Einfärbung und Einsatz von mikroskopischen Geräten können auch rheologische Techniken verwendet werden, um etwa eine medizinische Diagnostik zu betreiben. Diese Techniken können beispielsweise Rotationsrheometer oder Kapillarrheometer umfassen. Auch können Kugelfallviskosimeter oder mikrofluidische Vorrichtungen eingesetzt werden. Pathologisches Gewebe unterscheidet sich vom physiologischen Zustand beispielsweise durch seine viskosen, viskoelastischen oder elastischen Eigenschaften.

Beispielsweise lassen sich auf diese Weise Stenosen oder vergleichbare Leiden näher untersuchen. Körperflüssigkeiten, wie etwa Speichel, Blut et cetera, unterscheiden sich ebenfalls in ihren viskosen, viskoelastischen oder elastischen Eigenschaften, wenn sich eine Veränderung einstellt. Dieser Umstand ist zum Beispiel im Zusammenhang mit der Sichelzellenanämie bekannt. Insofern lassen sich Kenntnisse über rheologische Eigenschaften von physiologischen oder pathologischen Eigenschaften zur Diagnostik verwenden.

Dabei können Messungen von einer oder mehrerer rheologischen Größen, wie beispielsweise (Scher-)Viskosität, Dehnviskosität, Schubspannung, viskoelastische Eigenschaften, Speichermodul, Verlustmodul gemessen werden. Solche Messungen können absolute oder relative Messwerte umfassen. Typischerweise wird eine jeweilige Probe hierzu in Oszillation, Schwingung versetzt. Alternativ kann auch eine Rotation oder eine Bewegung in Form einer Strömung vorgesehen sein. Auch körpergegebene Umstände von Proben können bereits Formen von Oszillation, Schwingung, Rotation oder Strömungen bedingen, sodass dieser Umstand für rheologische Verfahren genutzt werden kann.

Die skizzierten Vorgehensweisen mit den jeweiligen einzusetzenden Techniken gehen mit einem gewissen Zeitaufwand einher. Zudem wird für den Transport der Proben eine bestimmte Logistikinfrastruktur benötigt, um die häufig sensiblen Proben während dieser Zeit nicht zu beschädigen. In diesem Zusammenhang sind trotz aller Vorsichtsmaßnahmen Veränderungen der Proben wahrscheinlich und können nicht gänzlich ausgeschlossen werden. Generell ändert sich biologisches Probenmaterial zeitlich schnell, sodass nachfolgende Untersuchungen beziehungsweise die Analytik erschwert wird. Weiterhin besteht ein Risiko, sich bei der Entnahme der Proben oder beim Transport der Proben, insbesondere bei biologischen Proben, einer ungewollten Infektion, wenn es zu einem entsprechenden Kontakt mit diesen Proben bei den zuvor genannten Aktivitäten kommt.

Für die oben erwähnten Fälle der Biopsie und der Entnahme während einer Operation ist die nötige medizinische Infrastruktur häufig nur bei Maximalversorgern gegeben. Eine Versorgung und Diagnostik in entlegenen Gegenden, wie beispielsweise im Hochgebirge oder auf hoher See, kann sich somit als schwierig erweisen, da die nötige medizinische Infrastruktur entweder nur schwer zu bereitstellbar oder schlicht nicht gegeben ist.

### ZUSAMMENFASSUNG DER ERFINDUNG

Vor diesem Hintergrund liegt der vorliegenden Erfindung die Aufgabe zugrunde, eine mobile Rheometervorrichtung und ein Verfahren bereitzustellen, welche zumindest teilweise die zuvor genannten Nachteile überkommen.

Diese Aufgabe wird gelöst durch eine mobile Rheometervorrichtung mit den Merkmalen des Patentanspruchs 1 sowie durch eine Verfahren mit den Merkmalen des Patentanspruchs 10.

Dementsprechend ist eine mobile Rheometervorrichtung für die medizinische Diagnostik von menschlichen, tierischen oder pflanzlichen Proben vorgesehen. Solch eine mobile Rheometervorrichtung umfasst dabei eine rheologische Messeinrichtung, eine damit verbundene Auswerteeinheit mit Auswerteprogramm und eine damit verbundene Ausgabeeinheit. Dabei ist die Auswerteeinheit mit Auswerteprogramm ausgelegt, die gemessenen rheologischen Parameter einer menschlichen, tierischen oder pflanzlichen Probe hinsichtlich wenigstens einer medizinischen Diagnose oder in relevante Parametergruppen für eine medizinische Diagnose zu klassifizieren, sodass einem Anwender der mobilen Rheometervorrichtung eine differenzierte Ansicht der Probe mittels der Ausgabeeinheit bereitstellbar ist.

Des Weiteren ist ein Verfahren für die medizinische Diagnostik von menschlichen, tierischen oder pflanzlichen Proben vorgesehen. Solch ein Verfahren umfasst dabei die folgenden Schritte: Bereitstellen und Aktivieren einer erfindungsgemäßen mobilen Rheometervorrichtung; Messen einer menschlichen, tierischen oder pflanzlichen Proben mittels der mobilen Rheometervorrichtung; Klassifizieren von Ergebnissen der rheologischen Messungen hinsichtlich wenigstens einer medizinischen Diagnose mittels der mobilen Rheometervorrichtung; Ausgeben der klassifizierten Ergebnisse mittels der mobilen Rheometervorrichtung, sodass einem Anwender der mobilen Rheometervorrichtung eine differenzierte Ansicht der Probe mittels der mobilen Rheometervorrichtung bereitstellbar ist.

Eine der Erfindung zugrunde liegende Idee besteht darin, eine mobile technische Lösung bereitzustellen, welche vor Ort direkt so eingesetzt werden kann, sodass unmittelbar auf die Messergebnisse von zu analysierenden Proben zugegriffen werden kann. Die vorgestellte mobile Rheometervorrichtung bietet den Vorteil, dass sie leicht an dem jeweiligen Einsatzort bewegt werden kann, wobei aufgrund der speziell vorgesehenen Auswerteeinheit eine Klassifizierung von Messwerten unmittelbar vor Ort durchführbar ist. Somit können aufwendige Transporte und langwierige Untersuchungsmethoden vermieden werden. Insbesondere Proben, die sich zeitlich schnell verändern und somit mitunter für eine gewünschte Analytik unbrauchbar werden, können somit besser und folgerichtiger analysiert werden.

Durch den Wegfall einer notwendigen komplexen Infrastruktur eines Maximalversorgers kann eine pathologische beziehungsweise allgemein eine medizinische Diagnostik einer größeren Menge an Menschen zugänglich gemacht werden. Menschen, deren Krankheitsverlauf getrackt werden muss, können dies etwa zu Hause machen und gegebenenfalls remote (aus der Ferne) versorgt werden, sodass sie seltener oder nur bei Bedarf mit medizinischem Fachpersonal in Kontakt treten müssen. Auch für einen breiten Einsatz im Zusammenhang mit möglichst frühzeitigen Diagnosen und somit besseren Perspektiven hinsichtlich eines Heilungsprozesses oder allgemein einer Prävention ist die vorgestellte mobile Rheometervorrichtung mit Vorteil einsetzbar.

Insofern sind die mobile Rheometervorrichtung und das entsprechende Verfahren als Teil einer Ausstattung von Operationssälen und Arztpraxen sowie für den Einsatz bei mobilen medizinischen Versorgungseinrichtungen- oder -einheiten mit Vorteil einsetzbar. Auch ein individueller Einsatz von medizinischen Laien, etwa vom Patienten direkt, ist vorstellbar, wobei die mobile Rheometervorrichtung etwa wie ein persönlicher Alltagsgegenstand als mobile Lösung für medizinische Diagnoseaktivitäten oder dergleichen mit Vorteil eingesetzt werden kann.

Gemäß eines Ausführungsbeispiel der mobilen Rheometervorrichtung ist vorgesehen, dass die Auswerteeinheit mit Auswerteprogramm für die Zwecke der Klassifizierung mit wenigstens einer Datenbank für die medizinische Diagnostik von menschlichen, tierischen oder pflanzlichen Proben basierend auf rheologischen Parametern verbindbar ist oder wenigstens eine solche Datenbank umfasst.

Auf diese Weise ist eine besonders schnelle und umfassende Klassifizierung durchführbar. Auch lässt sich je nach eingesetzter Datenbank das Einsatzgebiet der vorgestellten mobilen Rheometervorrichtung ausweiten. Zudem erhöht sich somit die Wahrscheinlichkeit bei einem Einsatz in entlegeneren Gebieten, dass die Verwendung der erfindungsgemäßen mobilen Rheometervorrichtung für eine folgerichtige Analytik als ausreichend einzustufen ist.

Dabei können etwa die rheologischen Eigenschaften mit der Datenbank (oder mit mehreren Datenbanken) mit Informationen zu den rheologischen Eigenschaften physiologischer oder pathologischer Natur abgeglichen werden. Durch den Abgleich erlaubt die Rheometervorrichtung die Beschreibung eines Hinweises oder mehrerer Hinweise für die Diagnostik (oder für den professionellen medizinischen Anwender) in Bezug auf den Zustand der Probe. Auch können Hinweise allgemeiner Natur in Form von Parametern oder dergleichen abgeleitet werden, die auch für einen medizinischen Laien leicht verständlich und zugänglich sind.

Mittels der vorgestellten mobilen Rheometervorrichtung und dem entsprechenden Verfahren ist es somit möglich, rheologische Untersuchungen mit Bezug zu einer medizinische Diagnostik von menschlichen, tierischen oder pflanzlichen Proben in unmittelbarer räumlicher und zeitlicher Nähe durchzuführen. Die rheologischen Untersuchungen erlauben dabei etwa Messungen unter möglichst physiologischen Bedingungen, zum Beispiel hinsichtlich eines pH-Wertes oder hinsichtlich einer bestimmten Temperatur, einer Ionenkonzentration, in Bezug auf vorhandene Enzyme oder weiteren Parametern etwa von Körperflüssigkeiten.

Gemäß einer Weiterbildung der mobilen Rheometervorrichtung ist vorgesehen, dass das Auswerteprogramm mittels wenigstens einer benutzerdefinierten Eingabe anpassbar ist, sodass eine individualisierte Nutzung der jeweiligen Datenbank möglich ist.

Somit kann eine noch speziellere Auswerteroutine unmittelbar vor Ort ermöglicht werden. Das Auswerteprogramm ist dabei mit einfachsten Eingaben anpassbar, sodass mittels der vorgestellten mobilen Rheometervorrichtung eine noch bessere Nutzung der verbundenen Datenbanken möglich sein kann. Beispielsweise können somit Einflüsse von Faktoren bei den rheologischen Messwerten korrigiert werden, die nicht notwendigerweise oder alleinig mit einer möglichen diagnostizierten Krankheit einhergehen müssen, sodass anschließend ein leichterer Abgleich mit Informationen aus der Datenbank beziehungsweise der Datenbanken möglich sein kann. In diesem Zusammenhang sind auch gängige Algorithmen als optionaler Teil des Auswerteprogramms in Anlehnung an eine künstliche Intelligenz oder aber auch Algorithmen als optionaler Teil des Auswerteprogramms in Anlehnung an gängige Maschinenlernprogramme vorstellbar. Insbesondere kann vorgesehen sein, dass das Auswerteprogramm ausgelegt ist, eine Fülle von Eingaben so zu verarbeiten, sodass eine klare Zuordnung im Sinne der vorliegenden Erfindung, sprich eine möglichst eindeutige Klassifizierung hinsichtlich einer Diagnose beziehungsweise der für eine Diagnose relevanten Parameter gewährleistet werden kann.

Gemäß einer Weiterbildung der mobilen Rheometervorrichtung ist vorgesehen, dass die wenigstens eine benutzerdefinierte Eingabe ausgewählt ist aus: gendersensible Eingabe, Ernährungsangabe über menschlichen oder tierischen Probanden, Medikamenteneinnahme eines menschlichen oder tierischen Probanden, Düngemittelabgabe an pflanzlichen Probanden, Pflanzenschutzabgabe an pflanzlichen Probanden, wenigstens eine Information über korrelierende Einflüsse von Medikamenten und pathologischen Veränderungen bei wenigstens einem Organismus, Verdachtsdiagnosen, wenigstens eine Information aus einem Patientenregister aus wenigstens einem Land, wenigstens eine Information über eine Vorerkrankung des zu untersuchenden Organismus.

Diese jeweiligen Eingaben können somit dazu verwendet werden, Klassifizierungen und die dabei verwendeten Abgleiche mit der Datenbank oder den Datenbanken noch besser zu gestalten. Insbesondere gendersensible Eingaben können vorteilhaft dazu eingesetzt werden, gezieltere Aussagen beziehungsweise Klassifizierungen zu gestalten, da somit die Berücksichtigung von geschlechterspezifischen Unterschieden unmittelbar in den Auswerteprozess mit integriert werden kann.

Die wenigstens eine Information über korrelierende Einflüsse von Medikamenten und pathologischen Veränderungen bei wenigstens einem Organismus kann beispielsweise im Sinne der Erkenntnis vorliegen, wie sie in Indien zwischen dem Einsatz von Diclofenac bei Nutztieren und dem Geiersterben auftraten. Aufgrund der Abgabe von Diclofenac an Nutztieren gelang über die Aufnahme des Aas diese Medikament in den jeweiligen Organismus der dortigen Geierpopulation, was ein Geiersterben zur Folge hatte, da dieses Medikament für Geier unverträglich ist.

Gemäß eines Ausführungsbeispiels der mobilen Rheometervorrichtung ist vorgesehen, dass die rheologische Messeinrichtung ausgelegt ist, rheologische Parameter der menschlichen, tierischen oder pflanzlichen Proben separat oder am Organismus zu messen.

Somit resultiert eine besonders flexibel einsetzbare mobile Rheometervorrichtung. Die Vorrichtung kann entweder direkt am zu beprobenden Organismus eingesetzt werden, ohne dass dieser nennenswert dabei beeinträchtigt wird. Dies bietet den Vorteil, dass die Probe, sprich der zu untersuchende Bereich des Organismus, nicht zerstört werden muss, sondern nach der Untersuchung im Wesentlichen im gleichen Zustand vorhanden ist. Insbesondere bei tumorartigen Veränderungen von Gewebeabschnitten im menschlichen oder tierischen Körper ist dieser Umstand wünschenswert, um verändertes Zellgewebe nicht unbeabsichtigt in Kreislaufsysteme des übrigen Organismus einzubringen. Mit anderen Worten kann die vorgestellte mobile Rheometervorrichtung auch in oder am Gewebe, beispielsweise vor der eigentlichen Entnahme des Gewebes oder allgemein der Materialprobe, verwendet werden. Somit bleibt das Gewebe intakt und wird nur dann operativ entfernt, wenn sich dieser Umstand aufgrund der durchgeführten medizinischen Diagnose als notwendig erweist. Sinngemäß kann so bei einem pflanzlichen Organismus bestimmt werden, welche Teile und in welchem Umfang diese zu entfernen sind. Beispielsweise aufgrund eines Pilz- oder Schädlingsbefalls.

Alternativ kann die Vorrichtung in unmittelbarer Nähe des zu beprobenden Organismus platziert werden, sodass entnommene Proben sofort untersucht werden können. Beispielsweise kann es sich dabei um eine Gewebeprobe eines menschlichen oder tierischen Körpers handeln, welche während einer Biopsie oder einer Operation entnommen wird. Auch kann es sich um ein entnommenes Teil eines pflanzlichen Körpers beziehungsweise pflanzlichen Organismus handeln.

Gemäß eines Ausführungsbeispiels der mobilen Rheometervorrichtung ist vorgesehen, dass die mobile Rheometervorrichtung ein Energiesystem umfasst, welches ausgelegt ist, die mobile Rheometervorrichtung autark oder mittels eines externen Energieversorgungsnetzes mit elektrischer Energie zu versorgen.

Das Energiesystem kann etwa wiederaufladbare Akkumulator-Systeme oder dergleichen umfassen, sodass sowohl ein batteriebetriebener Modus als auch ein Netzmodus möglich sind. Die mobile Rheometervorrichtung kann somit mit Vorteil noch flexibler eingesetzt werden.

Gemäß einer Weiterbildung der mobilen Rheometervorrichtung ist vorgesehen, dass die mobile Rheometervorrichtung im Wesentlichen Außenmaterialien, insbesondere Edelstahl oder Titan, umfasst, welche für ein Sterilisationsverfahren, insbesondere Gammastrahlensterilisation, geeignet sind, sodass die mobile Rheometervorrichtung nach Durchlauf eines Sterilisierverfahrens in einem Operationssaal oder bereits bei der Herstellung oder bei einem Dienstleister für das Sterilisieren von Medizinprodukten einsetzbar ist.

Insofern kann die mobile Rheometervorrichtung hygienisch designt beziehungsweise ausgelegt sein und somit mit Vorteil in einer zwingend sterilen Umgebung, wie etwa einem Operationssaal oder dergleichen, eingesetzt werden. In diesem Zusammenhang sind Außenmaterialien insbesondere die Materialien und Bereiche der Vorrichtung, welche im Wesentlichen innenliegende Hauptkomponenten der Vorrichtung zur Außenwelt abschirmen oder zumindest anteilig einhausen. Auch eine komplette Einhausung kann dabei vorgesehen sein.

Gemäß eines Ausführungsbeispiels der mobilen Rheometervorrichtung ist vorgesehen, dass die mobile Rheometervorrichtung ausgelegt ist, eine Vielzahl von Proben simultan zu messen und zu klassifizieren.

Auf diese Weise kann die mobile Rheometervorrichtung noch effizienter eingesetzt werden, sodass Kosten pro jeweiliger Untersuchungsprobe reduziert werden können.

Gemäß eines Ausführungsbeispiels der mobilen Rheometervorrichtung ist vorgesehen, dass die mobile Rheometervorrichtung ausgelegt ist, mittels wenigstens einem mit der mobilen Rheometervorrichtung koppelbaren externen Gerät gesteuert zu werden. Solch ein externes Gerät kann beispielsweise ein Smartphone, ein Tablet oder ein ähnliches mobiles Endgerät sein.

Für diese Zwecke kann etwa das Auswerteprogramm um eine Form erweitert werden, welche in Form einer Applikation oder dergleichen auf dem externen Gerät installierbar ist. Das Auswerteprogramm kann also mit anderen Worten ausgelegt sein, diese Applikation bereitzustellen, und etwa mittels Download oder dergleichen auf das externe Gerät zu transferieren, sodass dann die mobile Rheometervorrichtung mittels des koppelbaren externen Geräts gesteuert werden kann. In diesem Zusammenhang ist auch vorstellbar, dass das Auswerteprogramm ausgelegt ist, diese Funktion in Verbindung oder mittels eines entsprechenden Cloud-Ansatzes bereitzustellen. Auch kann das externe Gerät zumindest anteilig als cloudbasierte technische Lösung vorgesehen sein.

Gemäß eines Ausführungsbeispiels des Verfahrens ist vorgesehen, dass folgende weitere Verfahrensschritte vorgesehen werden: Verbinden der Auswerteeinheit mit Auswerteprogramm von der mobilen Rheometervorrichtung mit wenigstens einer Datenbank für die medizinische Diagnostik von menschlichen, tierischen oder pflanzlichen Proben basierend auf rheologischen Parametern; Anpassen des Auswerteprogramms mittels wenigstens einer benutzerdefinierten Eingabe, sodass eine individualisierte Nutzung der verbundenen externen Datenbank möglich ist.

Die zuvor genannten Vorteile gelten, soweit übertragbar, auch für diese Variante des erfindungsgemäßen Verfahrens. Insbesondere kann das vorgestellte Verfahren mit Vorteil verwendet werden, um grundsätzlich eine gendersensible Auswertung zu ermöglichen, sodass eine Unterstützung einer medizinischen Forschung, die auf geschlechterspezifische Unterschiede Rücksicht nimmt, ermöglicht wird.

### KURZBESCHREIBUNG DER ZEICHNUNGEN

Im Folgenden wird die Erfindung unter Bezugnahme auf die Figuren der Zeichnungen erläutert. Von den Figuren zeigen:
- Fig. 1: eine schematische Darstellung einer mobilen Rheometervorrichtung nach einem Ausführungsbeispiel der vorliegenden Erfindung;
- Fig. 2: eine schematische Darstellung eines Anwenderszenarios einer mobilen Rheometervorrichtung nach einem Ausführungsbeispiel der vorliegenden Erfindung;
- Fig. 3: eine schematische Darstellung eines weiteren Anwenderszenarios einer mobilen Rheometervorrichtung nach einem Ausführungsbeispiel der vorliegenden Erfindung;
- Fig. 4: ein schematisches Flussdiagramm für ein Verfahren nach einem Ausführungsbeispiel der vorliegenden Erfindung;
- Fig. 5: eine schematische Darstellung eines weiteren Anwenderszenarios einer mobilen Rheometervorrichtung nach einem Ausführungsbeispiel der vorliegenden Erfindung;
- Fig. 6: eine schematische Darstellung eines weiteren Anwenderszenarios einer mobilen Rheometervorrichtung nach einem Ausführungsbeispiel der vorliegenden Erfindung;
- Fig. 7: eine schematische Darstellung eines weiteren Anwenderszenarios einer mobilen Rheometervorrichtung nach einem Ausführungsbeispiel der vorliegenden Erfindung.

In den Figuren bezeichnen dieselben Bezugszeichen gleiche oder funktionsgleiche Komponenten, soweit nichts Gegenteiliges angegeben ist.

### AUSFÜHRLICHE BESCHREIBUNG VON AUSFÜHRUNGSBEISPIELEN DER ERFINDUNG

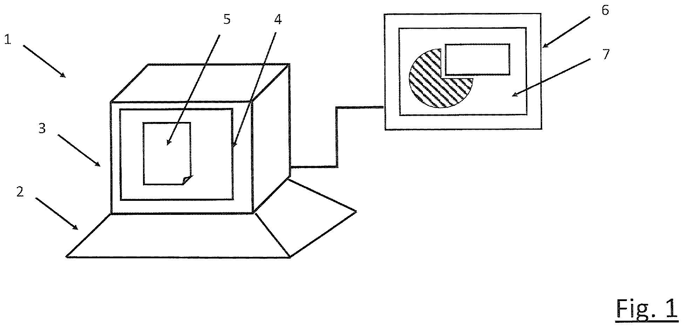

Fig. 1 zeigt eine schematische Darstellung einer mobilen Rheometervorrichtung 1 nach einem Ausführungsbeispiel der vorliegenden Erfindung. Insbesondere handelt es sich dabei um eine mobile Rheometervorrichtung 1 für die medizinische Diagnostik von menschlichen, tierischen oder pflanzlichen Proben. Bei den Proben kann es sich beispielsweise um menschliche Gewebeproben handeln.

Die mobile Rheometervorrichtung 1 ist dabei stark vereinfacht mit einer rheologischen Messeinrichtung 2 dargestellt, welch sich bezogen auf die Bildebene unterhalb eines Hauptkörpers 3 der mobilen Rheometervorrichtung 1 befindet. In nicht näher gezeigten Ausführungsvarianten der erfindungsgemäßen mobilen Rheometervorrichtung 1 ist vorstellbar, dass die rheologische Messeinrichtung 2 zumindest anteilig oder sogar gänzlich in dem Hauptkörper 3 angeordnet ist. Auch partiell seitliche Anordnungen oder eine komplette seitliche Anordnung sind vorstellbar. Auch ist vorstellbar, dass die rheologische Messeinrichtung 2 separat vorgesehen ist und mit dem Hauptkörper 3 und den dort sich befindlichen Komponenten mittels einer kabelgebundenen oder drahtlosen Verbindung gekoppelt ist. In einer weiteren Ausführungsform ist demnach auch denkbar, dass die Auswerteeinheit 4 mit dem Auswerteprogramm 5 zusätzlich (im Fall des Vorhandenseins von drahtlosen Verbindungstechniken oder dergleichen) oder allgemein in einem mobilen Endgerät vorgehalten werden oder dort installiert oder dort angeordnet oder dort allgemein vorgehalten oder dort mittels einer technischen Ergänzung, virtuell oder sogar physisch als separate Subeinheit der mobilen Rheometervorrichtung 1, vorgesehen beziehungsweise dementsprechend angeordnet sind.

In dem Hauptkörper 3 der mobilen Rheometervorrichtung 1 ist eine Auswerteeinheit 4 mit Auswerteprogramm 5 dargestellt. Diese Auswerteeinheit 4 mit Auswerteprogramm 5 ist mit der rheologischen Messeinrichtung 2 über nicht näher dargestellte Verbindungsmittel verbunden, sodass jeweilige mittels der rheologischen Messeinrichtung 2 gemessenen Messwerte an die Auswerteeinheit 4 mit Auswerteprogramm 5 übertragbar sind.

Die Auswerteeinheit 4 mit Auswerteprogramm 5 ist dabei ausgelegt, die gemessenen rheologischen Parameter einer menschlichen, tierischen oder pflanzlichen Probe hinsichtlich wenigstens einer medizinischen Diagnose zu klassifizieren, sodass einem Anwender der mobilen Rheometervorrichtung 1 eine differenzierte Ansicht der Probe mittels einer dargestellten und mit der Auswerteeinheit 4 mit Auswerteprogramm 5 verbundenen Ausgabeeinheit 6 bereitstellbar ist.

Die dargestellte Ausgabeeinheit 6 ist dabei im Wesentlichen in Form eines Monitors mit einer für die Darstellung von klassifizierten Ergebnissen geeigneten Anzeigefläche 7 vorgesehen. Diese Anzeigefläche 7 kann dabei ausgelegt sein, farbige Bilder in zweidimensionaler oder dreidimensionaler Ansicht bereitzustellen, was hier in Fig. 1 schematisch mit einem ¾ Kreissymbol und einem Rechteck verdeutlicht wird.

Dabei kann das ¾ Kreissymbol beispielhaft stellvertretend für gesundes Gewebe und das Rechteck für auffälliges Gewebe basierend auf der Klassifizierung angesehen werden. Die Ausgabeeinheit 6 ist über eine erste Verbindungsleitung 8 mit dem Hauptkörper 3 der mobilen Rheometervorrichtung 1 und somit mit der Auswerteeinheit 4 mit Auswerteprogramm 5 gekoppelt dargestellt, sodass einem Anwender der mobilen Rheometervorrichtung 1 eine differenzierte Ansicht der Probe mittels der Ausgabeeinheit 6 bereitstellbar ist.

Optional kann die mobile Rheometervorrichtung 1 ausgelegt sein, für eine Vielzahl simultan auszuwertender Proben genutzt zu werden, um beispielsweise die statistische Aussagekraft der Untersuchung zu verbessern.

Die nicht näher dargestellte wenigstens eine Datenbank kann mittels jeglicher Verbindungsmittel, beispielsweise kabelbasiert oder auch mittels Funktechnologie, mit der mobilen Rheometervorrichtung 1 gekoppelt werden. Auch ist vorstellbar, dass die wenigstens eine nicht näher dargestellte Datenbank zumindest anteilig ein Teil der mobilen Rheometervorrichtung 1 ist. Beispielsweise kann die nicht näher dargestellte wenigstens eine Datenbank somit zumindest anteilig ein Teilbereich der Auswerteeinheit 4 mit Auswerteprogramm 5 sein. Auch ist vorstellbar, dass die nicht näher dargestellte wenigstens eine Datenbank eine separate Einheit der mobilen Rheometervorrichtung 1 ist, wobei sie entsprechend gekoppelt mit den anderen Komponenten der mobilen Rheometervorrichtung 1 in dieser angeordnet vorgesehen ist.

Die nicht näher dargestellte wenigstens eine Datenbank kann somit entweder ein fester Bestandteil der mobilen Rheometervorrichtung 1 sein oder mit dieser über Schnittstellen der mobilen Rheometervorrichtung 1 verknüpft sein. In beiden Fällen kann somit die wenigstens eine Datenbank medizinischem Fachpersonal direkt während oder nach der rheologischen Messung eine Auswertung der rheologischen Parameter und einer damit verbundenen Diagnose ermöglichen.

Die wenigstens eine Datenbank kann, wie bereits erwähnt, mit weiteren Eingaben, sprich weiteren Daten, wie zum Beispiel Angaben über eine Ernährung oder einer Medikation des Patienten versehen werden, sodass somit Korrelationen und Korrekturen vornehmbar sind, welche mit diesen neuen Eingaben eine individualisierte Form der wenigstens eine Datenbank ermöglichen. Auf diese Weise können beispielsweise Einflüsse von Faktoren korrigiert werden, die nicht notwendigerweise oder alleinig mit der diagnostizierten Krankheit einhergehen müssen. Dabei kann die mobile Rheometervorrichtung 1 gendersensibel auswerten und somit eine medizinische Forschung unterstützen, die auf geschlechterspezifische Unterschiede Rücksicht nimmt.

Speziell gestaltete Geometrien der mobilen Rheometervorrichtung 1, welche beispielsweise Einweggeometrien sein können, können dabei vorgesehen sein, sodass eine standardisierte Probenaufgabe auf der mobilen Rheometervorrichtung 1 möglich ist.

Die mobile Rheometervorrichtung 1 kann in einer nicht näher dargestellten Ausführungsform über einen Mechanismus verfügen, beispielsweise in Form einer Software oder dergleichen in Form etwa einer Applikation, welche die Prozedur der Probennahme, des Probeaufbringens, Messens und Auswerten, insbesondere der Klassifizierung, so sehr standardisiert, sodass interpersonelle Unterschiede aufgrund des Bedienpersonals oder eines Bedienenden minimierbar sind.

Wie bereits erwähnt, kann die mobile Rheometervorrichtung 1 portabel ausgelegt sein. Die mobile Rheometervorrichtung 1 kann Netz- sowie Batteriebetrieben verwendet werden, um sowohl im stationären Betrieb wie auch in einem mobilen Betrieb verwendet werden zu können. Beispielsweise kann somit die mobile Rheometervorrichtung 1 in entlegenen Gegenden autark betrieben werden. Insofern ist die vorgesehene mobile Rheometervorrichtung 1 ein medizinisches portables Gerät, welches für einen weitestgehend autonomen Betrieb auch in entlegeneren Gegenden ohne eine ausgeprägte Infrastruktur zum Einsatz kommen kann.

Die mobile Rheometervorrichtung 1 kann am Körper eingesetzt werden, sodass die Probenahme ohne Eingriff im menschlichen, tierischen oder pflanzlichen Körper und somit auch von medizinischen Laien durchführbar ist. Der Fokus kann bei einer solchen Vornahme beziehungsweise Anwendung auf intrapersonellen Trends liegen und es können dabei lediglich Indikatoren kommuniziert werden, sprich keine Diagnosen, die nur medizinischem Fachpersonal vorbehalten wären.

Die mobile Rheometervorrichtung 1 kann in seiner Bauform, insbesondere hinsichtlich seiner räumlichen Maße, ein Handgerät sein. Insofern kann die mobile Rheometervorrichtung 1 in Form eines kleineren Gerätes vorgesehen sein, welches komfortabel und ohne größere Anstrengungen in einer Hand eines Anwenders gehalten werden kann. Der Vorteil besteht somit weiter darin, dass die vorgestellte mobile Rheometervorrichtung 1 ausgelegt ist, eine leichte Ausführung zu ermöglichen, sodass ein mobiler Einsatz neben einer stationären Ausführung möglich ist.

Weiter kann die mobile Rheometervorrichtung 1 in einer nicht näher dargestellten Ausführungsform ausgelegt sein, mit einem Smartphone, Tablet oder einem ähnlichem mobilen Endgerät oder aus einer Cloud heraus gesteuert zu werden.

Die mobile Rheometervorrichtung 1 kann als ein spezielles Rheometer angesehen beziehungsweise so bezeichnet werden, welche zur Sofort-Analyse für medizinisches Fachpersonal und medizinische Laien in Verbindung mit einer pathologischen Datenbank ausgelegt ist.

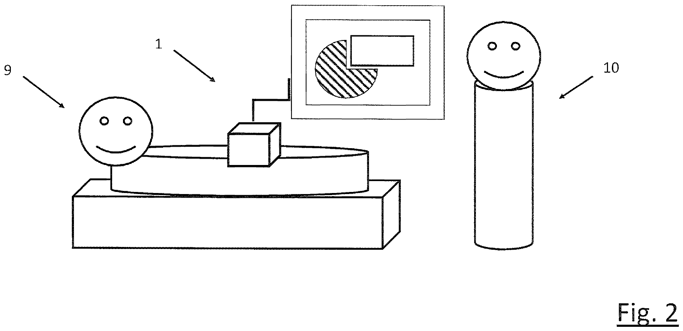

Fig. 2 zeigt eine schematische Darstellung eines Anwenderszenarios einer mobilen Rheometervorrichtung 1 nach einem Ausführungsbeispiel der vorliegenden Erfindung. Es kann sich beispielsweise um eine mobile Rheometervorrichtung 1 gemäß Figur 1 handeln.

Die mobile Rheometervorrichtung 1 ist dabei direkt im Kontakt mit einem Patienten 9 dargestellt. Dabei ist vorstellbar, dass die mobile Rheometervorrichtung 1 beziehungsweise die zugehörige rheologische Messeinrichtung 2 zumindest teilweise direkt im Körper des Patienten 9 oder nur am Körper des Patienten 9 angeordnet vorgesehen ist. Mit anderen Worten kann die Diagnostik entweder direkt im oder am Körper durchgeführt werden, wobei die mobile Rheometervorrichtung 1 mit ihren entsprechenden Komponenten für beide Varianten ausgelegt ist.

In einer Variante kann beispielsweise vorgesehen sein, dass die mobile Rheometervorrichtung 1 am Körper des Patienten 9 vor der Entnahme einer Gewebeprobe vorgesehen ist, sodass diese Probe dann direkt von der mobilen Rheometervorrichtung 1 entsprechend diagnostiziert werden kann, wenn sie entnommen ist.

Bezogen auf die Bildebene ist rechts ein Anwender 10 der mobilen Rheometervorrichtung 1 dargestellt. Es kann sich dabei etwa um medizinisches Personal, beispielsweise eine Operateurin, handeln.

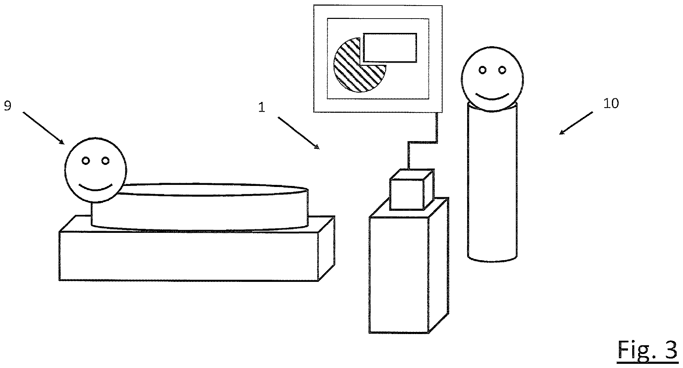

Fig. 3 zeigt eine schematische Darstellung eines weiteren Anwenderszenarios einer mobilen Rheometervorrichtung 1 nach einem Ausführungsbeispiel der vorliegenden Erfindung. Es kann sich beispielsweise um eine mobile Rheometervorrichtung 1 gemäß Figur 1 handeln.

Die mobile Rheometervorrichtung 1 ist dabei nicht direkt im Kontakt mit einem Patienten 9 dargestellt, sondern in räumlicher Nähe zu dem Patienten 9 platziert. Beispielsweise ist die mobile Rheometervorrichtung 1 im gleichen Operationssaal wie der der Patient 9 vorgesehen, sodass die mobile Rheometervorrichtung 1 während der Operation, sprich in räumlicher Nähe zum zu untersuchenden Körper des Patienten 9, vorgesehen ist.

Insofern kann die mobile Rheometervorrichtung 1 in räumlicher Nähe vorgesehen sein, sodass sie unmittelbar nach einer Entnahme einer Probe, beispielsweise einer Gewebeprobe, zum Einsatz kommen kann.

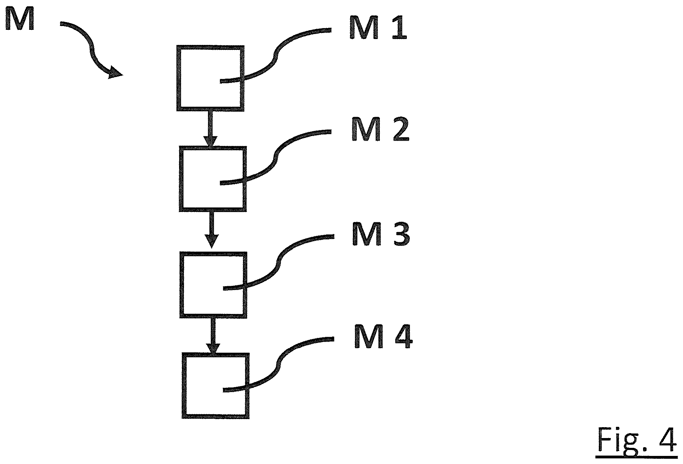

Fig. 4 zeigt ein schematisches Flussdiagramm für ein Verfahren M für die medizinische Diagnostik von menschlichen, tierischen oder pflanzlichen Proben nach einem Ausführungsbeispiel der vorliegenden Erfindung.

In einem ersten Verfahrensschritt M1 wird eine erfindungsgemäße mobile Rheometervorrichtung bereitgestellt und aktiviert. In einem zweiten Verfahrensschritt M2 werden menschliche, tierische oder pflanzliche Proben mittels der erfindungsgemäßen mobilen Rheometervorrichtung gemessen.

In einem dritten Verfahrensschritt M3 werden Ergebnisse der rheologischen Messungen hinsichtlich wenigstens einer medizinischen Diagnose mittels der erfindungsgemäßen mobilen Rheometervorrichtung klassifiziert. In einem vierten Verfahrensschritt M4 werden die klassifizierten Ergebnisse mittels der erfindungsgemäßen mobilen Rheometervorrichtung ausgegeben, sodass einem Anwender der mobilen Rheometervorrichtung eine differenzierte Ansicht der Probe mittels der mobilen Rheometervorrichtung bereitstellbar ist.

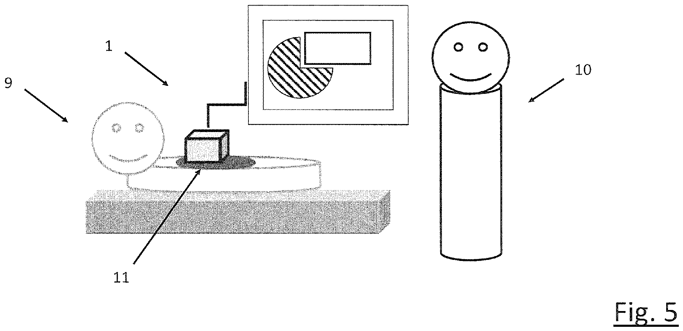

Fig. 5 zeigt eine schematische Darstellung eines weiteren Anwenderszenarios einer mobilen Rheometervorrichtung 1 nach einem Ausführungsbeispiel der vorliegenden Erfindung. Es kann sich beispielsweise um eine mobile Rheometervorrichtung 1 gemäß Figur 1 handeln.

Die mobile Rheometervorrichtung 1 ist dabei direkt im Kontakt mit einem Patienten 9 dargestellt. Dabei ist vorstellbar, dass die mobile Rheometervorrichtung 1 beziehungsweise die zugehörige rheologische Messeinrichtung 2 zumindest teilweise direkt im Körper des Patienten 9 oder nur am Körper des Patienten 9 angeordnet vorgesehen ist. Mit anderen Worten kann die Diagnostik entweder direkt im oder am Körper durchgeführt werden, wobei die mobile Rheometervorrichtung 1 mit ihren entsprechenden Komponenten für beide Varianten ausgelegt ist.

Im Gegensatz zur Darstellung in Fig. 2 ist in Bauchhöhe des Patienten 9 eine geöffnete Stelle 11 seines Körpers dargestellt. Mit anderen Worten wird hier der Körper des Patienten 9 geöffnet und anschließend wird die Probe charakterisiert beziehungsweise an der geöffneten Stelle 11 seines Körpers gemessen. Dies kann beispielsweise durch mechanisches Kontaktieren eines festkörperartigen Gewebes, Fluids oder eines Verdauungs-Zwischen- oder Endprodukts oder dergleichen vollzogen werden.

Bezogen auf die Bildebene ist rechts ein Anwender 10 der mobilen Rheometervorrichtung 1 dargestellt. Es kann sich dabei etwa um medizinisches Personal, beispielsweise eine Operateurin, handeln.

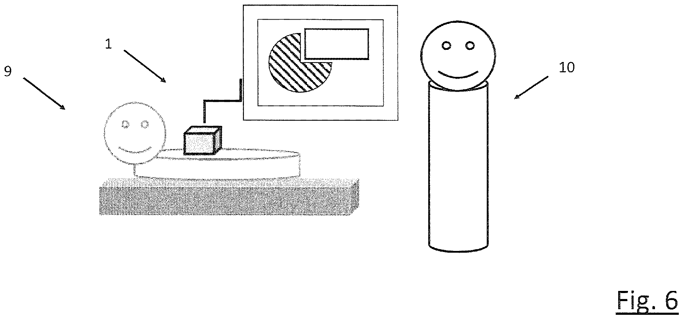

Fig. 6 zeigt eine schematische Darstellung eines weiteren Anwenderszenarios einer mobilen Rheometervorrichtung 1 nach einem Ausführungsbeispiel der vorliegenden Erfindung. Es kann sich beispielsweise um eine mobile Rheometervorrichtung 1 gemäß Figur 1 handeln.

Die mobile Rheometervorrichtung 1 ist dabei direkt im Kontakt mit einem Patienten 9 dargestellt. Dabei ist vorstellbar, dass die mobile Rheometervorrichtung 1 beziehungsweise die zugehörige rheologische Messeinrichtung 2 zumindest teilweise direkt im Körper des Patienten 9 oder nur am Körper des Patienten 9 angeordnet vorgesehen ist. Mit anderen Worten kann die Diagnostik entweder direkt im oder am Körper durchgeführt werden, wobei die mobile Rheometervorrichtung 1 mit ihren entsprechenden Komponenten für beide Varianten ausgelegt ist.

Im Gegensatz zur Darstellung in Fig. 5 ist in Bauchhöhe des Patienten 9 keine geöffnete Stelle seines Körpers dargestellt.

Die mobile Rheometervorrichtung 1 beziehungsweise die zugehörige rheologische Messeinrichtung 2 wird auf den Körper des Patienten 9 lediglich oberflächlich aufgebracht. Dies kann beispielsweise mit einer geringen Normalkraft vollzogen werden. Mit anderen Worten wird die mobile Rheometervorrichtung 1 beziehungsweise die zugehörige rheologische Messeinrichtung 2 mit geringer Normalkraft gehalten. Anschließend kann beispielsweise dann eine oszillatorische Messung von zum Beispiel Hautgewebe durchgeführt werden.

Bezogen auf die Bildebene ist rechts ein Anwender 10 der mobilen Rheometervorrichtung 1 dargestellt. Es kann sich dabei etwa um medizinisches Personal, beispielsweise eine Operateurin, handeln.

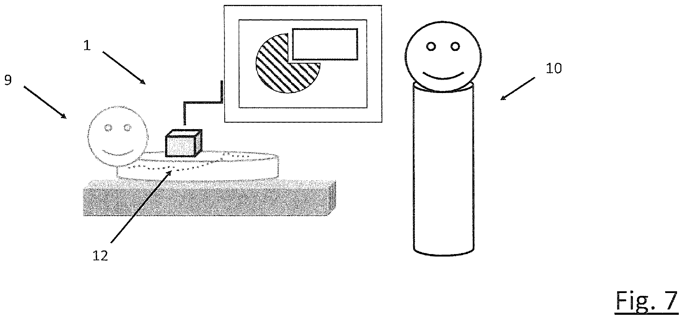

Fig. 7 zeigt eine schematische Darstellung eines weiteren Anwenderszenarios einer mobilen Rheometervorrichtung 1 nach einem Ausführungsbeispiel der vorliegenden Erfindung. Es kann sich beispielsweise um eine mobile Rheometervorrichtung 1 gemäß Figur 1 handeln.

Die mobile Rheometervorrichtung 1 ist dabei direkt im Kontakt mit einem Patienten 9 dargestellt. Dabei ist vorstellbar, dass die mobile Rheometervorrichtung 1 beziehungsweise die zugehörige rheologische Messeinrichtung 2 zumindest teilweise direkt im Körper des Patienten 9 oder nur am Körper des Patienten 9 angeordnet vorgesehen ist. Mit anderen Worten kann die Diagnostik entweder direkt im oder am Körper durchgeführt werden, wobei die mobile Rheometervorrichtung 1 mit ihren entsprechenden Komponenten für beide Varianten ausgelegt ist.

Im Gegensatz zur Darstellung in Fig. 5 ist in Bauchhöhe des Patienten 9 keine geöffnete Stelle seines Körpers dargestellt. Die mobile Rheometervorrichtung 1 beziehungsweise die zugehörige rheologische Messeinrichtung 2 wird auf den Körper des Patienten 9 lediglich oberflächlich aufgebracht.

Vor den eigentlichen rheologischen Messungen wird ein Körpergewebe oder eine Flüssigkeit des Patienten 9 in diesem Bereich mit einem Kontrastmittel eingefärbt. Mittels tomographischer Methoden oder dergleichen wird anschließend etwa über das Fließverhalten oder durch ein viskoelastisches Verhalten dann wenigstens ein Ergebnis ermittelt. Beim Fließverhalten kann etwa ein Strömungsprofil ermittelt werden. In der Fig. 7 wird dies anhand eines schematisch grob skizzierten Blutgefäß 12, in welchem entsprechend eingefärbtes Blut fließt, dargestellt.

Insofern ist vorstellbar, dass mittels der mobilen Rheometervorrichtung 1 beziehungsweise mittels der zugehörigen rheologischen Messeinrichtung 2 ein Strömungsprofil von Blut in Blutgefäßen 12 ermittelt werden kann. Viskoelastische Verhaltensweisen von Proben können mittels der mobilen Rheometervorrichtung 1 beziehungsweise die zugehörige rheologische Messeinrichtung 2 ermittelt werden, indem beispielsweise Gewebestrukturen oder allgemein Strukturen der Probe durch ein aufgebautes, insbesondere ein temporär aufgebautes, elektromagnetisches Feld, welches aufgebracht wird, in Bewegung gebracht werden, sodass dann eine entsprechende rheologische Charakteristik ermittelt werden kann.

Bezogen auf die Bildebene ist rechts ein Anwender 10 der mobilen Rheometervorrichtung 1 dargestellt. Es kann sich dabei etwa um medizinisches Personal, beispielsweise eine Operateurin, handeln.

In einer nicht näher dargestellten Ausführungsform ist vorstellbar, dass die Probe des Patienten 9, zum Beispiel Synovialflüssigkeit, Blut, ein Perikard-Fluid, Liquor cerebrospinalis, aus dem Körper für die Untersuchung entnommen wird und anschließend dem Körper wieder zugeführt wird. Die mobile Rheometervorrichtung 1 beziehungsweise die zugehörige rheologische Messeinrichtung 2 können für diese Zwecke speziell ausgelegt sein.

Beispielsweise können die mobile Rheometervorrichtung 1 beziehungsweise die zugehörige rheologische Messeinrichtung 2 Mittel umfassen, um einen ankommenden Schlauch mit der im Wesentlichen flüssigen Probe aufzunehmen, durch die mobile Rheometervorrichtung 1 beziehungsweise die zugehörige rheologische Messeinrichtung 2 hindurchzuleiten und anschließend wieder über einen weiteren Schlauch zu dem Körper des Patienten 9 zurückzuführen. Statt eines Schlauchs können auch andere Transportvorrichtungen zum Einsatz kommen, wobei ein Schlauch, welcher beispielsweise im Wesentlichen aus einem Kunststoff oder dergleichen gefertigt ist, vorteilhaft ist, da er sich für die oben genannten Aktionen flexibel im Raum biegen lässt, sodass eine komfortable Vorrichtung 1 resultiert.

In diesem Zusammenhang ist auch vorstellbar, dass die entnommene Flüssigkeit beziehungsweise die im Wesentlichen flüssige Probe anschließend nicht wieder dem Körper zugeführt wird, sondern entsprechend den geltenden Regeln entsorgt wird.

### BEZUGSZEICHENLISTE

- 1: mobile Rheometervorrichtung
- 2: rheologische Messeinrichtung
- 3: Hauptkörper
- 4: Auswerteeinheit
- 5: Auswerteprogramm
- 6: Ausgabeeinheit
- 7: Anzeigefläche
- 8: erste Verbindungsleitung
- 9: Patient
- 10: Anwender
- 11: geöffnete Stelle
- 12: Blutgefäß
- M: Verfahren
- M1: erster Verfahrensschritt
- M2: zweiter Verfahrensschritt
- M3: dritter Verfahrensschritt
- M4: vierter Verfahrensschritt

## Patentansprüche

1. Mobile Rheometervorrichtung (1) für die medizinische Diagnostik von menschlichen, tierischen oder pflanzlichen Proben umfassend eine rheologische Messeinrichtung (2), eine damit verbundene Auswerteeinheit (4) mit Auswerteprogramm (5) und eine damit verbunden Ausgabeeinheit (6) **dadurch gekennzeichnet, dass** die Auswerteeinheit (4) mit Auswerteprogramm (5) ausgelegt ist, die gemessenen rheologischen Parameter einer menschlichen, tierischen oder pflanzlichen Probe hinsichtlich wenigstens einer medizinischen Diagnose oder in relevante Parametergruppen für eine medizinische Diagnose zu klassifizieren, sodass einem Anwender der mobilen Rheometervorrichtung (1) eine differenzierte Ansicht der Probe mittels der Ausgabeeinheit (6) bereitstellbar ist.

2. Mobile Rheometervorrichtung (1) nach Anspruch 1, wobei die Auswerteeinheit (4) mit Auswerteprogramm (5) für die Zwecke der Klassifizierung mit wenigstens einer Datenbank für die medizinische Diagnostik von menschlichen, tierischen oder pflanzlichen Proben basierend auf rheologischen Parametern verbindbar ist oder wenigstens eine solche Datenbank umfasst.

3. Mobile Rheometervorrichtung (1) nach Anspruch 2, wobei das Auswerteprogramm (5) mittels wenigstens einer benutzerdefinierten Eingabe anpassbar ist, sodass eine individualisierte Nutzung der jeweiligen Datenbank möglich ist.

4. Mobile Rheometervorrichtung (1) nach Anspruch 3, wobei die wenigstens eine benutzerdefinierte Eingabe ausgewählt ist aus: gendersensible Eingabe, Ernährungsangabe über menschlichen oder tierischen Probanden, Medikamenteneinnahme eines menschlichen oder tierischen Probanden, Düngemittelabgabe an pflanzlichen Probanden, Pflanzenschutzabgabe an pflanzlichen Probanden, wenigstens eine Information über korrelierende Einflüsse von Medikamenten und pathologischen Veränderungen bei wenigstens einem Organismus, Verdachtsdiagnosen, wenigstens eine Information aus einem Patientenregister aus wenigstens einem Land, wenigstens eine Information über eine Vorerkrankung des zu untersuchenden Organismus.

5. Mobile Rheometervorrichtung (1) nach einem der vorhergehenden Ansprüche, wobei die rheologische Messeinrichtung (2) ausgelegt ist, rheologische Parameter der menschlichen, tierischen oder pflanzlichen Proben separat oder am Organismus zu messen.

6. Mobile Rheometervorrichtung (1) nach einem der vorhergehenden Ansprüche, wobei die mobile Rheometervorrichtung (1) ein Energiesystem umfasst, welches ausgelegt ist, die mobile Rheometervorrichtung (1) autark oder mittels eines externen Energieversorgungsnetzes mit elektrischer Energie zu versorgen.

7. Mobile Rheometervorrichtung (1) nach einem der vorhergehenden Ansprüche, wobei die mobile Rheometervorrichtung (1) im Wesentlichen Außenmaterialien, insbesondere Edelstahl oder Titan, umfasst, welche für ein Sterilisationsverfahren, insbesondere Gammastrahlensterilisation, geeignet sind, sodass die mobile Rheometervorrichtung (1) nach Durchlauf eines Sterilisierverfahrens in einem Operationssaal oder bereits bei der Herstellung oder bei einem Dienstleister für das Sterilisieren von Medizinprodukten einsetzbar ist.

8. Mobile Rheometervorrichtung (1) nach einem der vorhergehenden Ansprüche, wobei die mobile Rheometervorrichtung (1) ausgelegt ist, eine Vielzahl von Proben simultan zu messen und zu klassifizieren.

9. Mobile Rheometervorrichtung (1) nach einem der vorhergehenden Ansprüche, wobei die mobile Rheometervorrichtung (1) ausgelegt ist, mittels wenigstens einem mit der mobilen Rheometervorrichtung (1) koppelbaren externen Gerät gesteuert zu werden.

10. Verfahren für die medizinische Diagnostik von menschlichen, tierischen oder pflanzlichen Proben umfassend die folgenden Schritte:
• Bereitstellen und Aktivieren einer mobilen Rheometervorrichtung (1) nach einem der Ansprüche 1 bis 9;
• Messen einer menschlichen, tierischen oder pflanzlichen Probe mittels der mobilen Rheometervorrichtung (1);
• Klassifizieren von Ergebnissen der rheologischen Messungen hinsichtlich wenigstens einer medizinischen Diagnose mittels der mobilen Rheometervorrichtung (1);
• Ausgeben der klassifizierten Ergebnisse mittels der mobilen Rheometervorrichtung (1), sodass einem Anwender der mobilen Rheometervorrichtung (1) eine differenzierte Ansicht der Probe mittels der mobilen Rheometervorrichtung (1) bereitstellbar ist.

11. Verfahren nach Anspruch 10, umfassend die folgenden weiteren Schritte:
• Verbinden der Auswerteeinheit (4) mit Auswerteprogramm (5) von der mobilen Rheometervorrichtung (1) mit wenigstens einer Datenbank für die medizinische Diagnostik von menschlichen, tierischen oder pflanzlichen Proben basierend auf rheologischen Parametern;
• Anpassen des Auswerteprogramms (5) mittels wenigstens einer benutzerdefinierten Eingabe, sodass eine individualisierte Nutzung der verbundenen externen Datenbank möglich ist.
